Europäisches Patentamt

European Patent Office

Office européen des brevets

(19)

(11) Numéro de publication: **0 427 579 B1**

(12) **FASCICULE DE BREVET EUROPEEN**

(45) Date de publication de fascicule du brevet: **24.05.95**

(51) Int. Cl.⁶: **C01B 39/26**, B01J 29/18, C07C 5/27, C07C 15/08

(21) Numéro de dépôt: **90402799.2**

(22) Date de dépôt: **09.10.90**

(54) **Zéolithe de type mordénite et son procédé de préparation.**

(30) Priorité: **10.10.89 FR 8913317**

(43) Date de publication de la demande:
**15.05.91 Bulletin 91/20**

(45) Mention de la délivrance du brevet:
**24.05.95 Bulletin 95/21**

(84) Etats contractants désignés:
**DE FR GB**

(56) Documents cités:
EP-A- 0 040 104    EP-A- 0 053 499
EP-A- 0 080 615    EP-A- 0 269 503
EP-A- 0 351 311    US-A- 3 597 155

PATENT ABSTRACTS OF JAPAN vol. 8, no.
134 (C-230)(1571), 21 juin 1984; & JP - A -
5946138 (MITSUI FLUORO CHEM) 15.03.1984

(73) Titulaire: **INSTITUT FRANCAIS DU PETROLE**
**4, Avenue de Bois Préau**
**F-92502 Rueil-Malmaison (FR)**

(72) Inventeur: **Caullet, Philippe**
**4, rue Kellermann**
**F-68110 Illzach (FR)**
Inventeur: **Guth, Jean-Louis**
**59, rue Bellevue Brunstatt**
**F-68200 Mulhouse (FR)**
Inventeur: **Faust, Anne-Catherine**
**23, rue de Bourgogne**
**F-68200 Mulhouse (FR)**
Inventeur: **Joly, Jean-Francois**
**20, Rue Béranger**
**F-75003 Paris (FR)**
Inventeur: **Travers, Christine**
**12, Bd du Général de Gaulle**
**F-92500 Rueil Malmaison (FR)**
Inventeur: **Raatz, Francis**
**10, Allée Jacques Prévert**
**F-78260 Acheres (FR)**

EP 0 427 579 B1

## Description

La présente invention concerne une nouvelle zéolithe de type mordénite et un procédé de préparation de cette zéolithe.

En raison de leurs propriétés de sélectivité géométrique et d'échange ionique, les zéolithes sont utilisées industriellement sur une large échelle aussi bien en adsorption (par exemple séchage de gaz, séparation de paraffines linéaires et branchées, séparation de composés aromatiques etc) qu'en catalyse (par exemple craquage catalytique, hydrocraquage, isomérisation, oligomérisation etc).

La composition chimique des zéolithes contenant dans leur charpente les éléments Si et Al peut être représentée par la formule approchée suivante :

$$M_{2/n}O, Al_2O_3, xSiO_2$$

où M représente un cation de valence n, tel que par exemple un alcalin, un alcalino-terreux ou un cation organique, x est susceptible de varier en fonction des structures entre deux et l'infini, auquel cas la zéolithe est une silice microporeuse.

Bien que de nombreuses zéolithes du type aluminosilicate existent dans la nature, la recherche de produits ayant des propriétés nouvelles a conduit à la synthèse d'une grande variété de ces aluminosilicates à structure zéolithique dont la mordénite fait partie. Rappelons à cet égard que la mordénite est une zéolithe qui cristallise dans le système orthorhombique avec des paramètres cristallins a, b, c proches respectivement de 18,1, 20,5 et 7,5 Angströms (1 Angström = 1 Å - $10^{-10}$ mètre). Son réseau poreux est monodimensionnel, l'ouverture des pores étant délimitée par des cycles à 12 oxygènes.

Une des premières synthèses de mordénite a été rapportée au début des années soixante (A. KEOUGH and L. SAND, J. Am. Chem. Soc, vol. 83, 1961, p. 3536) : cette synthèse est réalisée en milieu alcalin, le cation de compensation étant $Na^+$. Le rapport Si/Al de cette mordénite synthétique est voisin de la valeur connue pour la mordénite naturelle, c'est-à-dire 5. Contrairement aux mordénites d'origine naturelle, cette variété synthétique est du type à larges pores, c'est-à-dire qu'elle possède des propriétés d'adsorption qui sont en accord avec sa structure cristallographique. En milieu alcalin et en absence de structurants organiques, les rapports Si/Al maximaux sont voisins de 10 (O. WHITTEMORE, Am. Mineralogist, Vol. 57, 1972, p. 1146 ; P. BODART, J.B. NAGY, E. DEROUANE, Z. GABELICA, Stud. Surf. Sci. Catal., Vol. 18, 1984, p. 125). L'utilisation de cations ou de molécules organiques dans le mélange réactionnel alcalin a permis de synthétiser des mordénites de rapport Si/Al supérieur à 10. Il faut noter que dans ces synthèses en présence d'agents organiques, le cation sodium est toujours associé au milieu de synthèse. Parmi ces synthèses en présence de composés organiques on peut par exemple citer les brevets suivants : JP5888118, US-A-4052472, EP-A-80615, US-A-4525466.

Toutes les zéolithes de type mordénite préparées jusqu'à présent ont été synthétisées en milieu classique, c'est-à-dire dans un milieu alcalin à un pH généralement supérieur à 9, milieu dans lequel l'agent mobilisateur de la silice est l'anion $OH^-$. Un autre milieu de synthèse des zéolithes a été récemment découvert : il s'agit du milieu fluorure, dans lequel l'agent mobilisateur de la silice est l'anion $F^-$; dans ce milieu, le pH est généralement inférieur à 10 (voir par exemple J.L. GUTH, H. KESSLER and R. WEY, Proc. 7th. Int. Zeolite. Conf, Tokyo, août, 17-22, 1986, p. 121). La synthèse d'un nombre limité de structures zéolithiques a déjà été réussie dans ce nouveau milieu, comme par exemple la MFI (demande de brevet français n° 88/09631) et la ferriérite (demande de brevet français n° 86/16362)

Par rapport au milieu de synthèse alcalin ($OH^-$), le milieu fluorure présente un certain nombre d'avantages très appréciables. En effet, en milieu alcalin, la plupart des zéolithes synthétisées sont métastables : on risque donc au cours de la synthèse de voir apparaître des phases solides plus stables et de précipiter des phases non désirées. Cette difficulté ne fait que s'accroître lorsque les quantités à préparer augmentent, c'est-à-dire lorsque l'on passe du stade du laboratoire au stade industriel. Par ailleurs, ces zéolithes métastables dans le milieu réactionnel basique ne sont obtenues que grâce à une forte sursaturation en espèces actives dans le milieu. Ceci provoque une nucléation rapide et, par conséquent,conduit à des cristaux de petites tailles, les dimensions moyennes de ces cristaux se situant dans le domaine du micromètre. L'élaboration de cristaux de plus grande taille est donc difficile en milieu basique. Or, dans certaines applications, il peut être intéressant de disposer de cristaux de grande taille de manière à préserver par exemple la stabilité thermique du solide.

Par ailleurs, la chimie des espèces aluminosilicatées en milieu fluorure est tout à fait différente de celle connue dans le milieu alcalin classique. Les synthèses en milieu fluorure sont donc succeptibles de conduire à des solides dont la distribution Si-Al locale dans la charpente est différente de celle connue pour les zéolithes synthétisées en milieu classique. Ce point revêt une importance particulière dans le cas de la mordénite. En effet, l'aluminium est situé, pour les mordénites connues dans l'art antérieur, préférentiellement dans les cycles à 4 de la charpente (V. GRAMLICH, Ph.D, Dissertation ETH n° 4633, Zurich, 1971 ; G. DEBRAS, J.B. NAGY, Z. GABELICA, P. BODART, P.A. JACOBS, Chem. Lett, 1983, p. 199) ; l'appariement de 2 atomes d'aluminium dans chaque cycle est thermodynamiquement favorisé (E.G. DEROUANE, J.G. FRIPIAT, Proc. 6th Int. Zeolite Conf., Reno, USA, 1984, p 717). Cette situation présente deux inconvénients : d'une part, la proximité des atomes d'aluminium a tendance à limiter la force des sites acides des formes H et, d'autre part, la présence de telles paires Al-Al, même dans les formes H désaluminées, se traduit par des sélectivités médiocres dans certaines réactions pour lesquelles les réactions bi-sites sont à proscrire (exemple de l'isomérisation des C8 aromatiques). Préparer des mordénites à teneur réduite en paires Al-Al apparait donc comme une voie d'innovation particulièrement intéressante.

D'autre part, la demande de brevet EP-A-0.053.499 décrit un procédé de préparation, par réaction hydrothermale et en présence d'un compose fluoré, d'une zéolithe qui apparaît être de la MFI, à partir d'une solution de départ comprenant au moins deux colloïdes ayant différents rapports Si/Al. Le document "Patent Abstracts of Japan vol. 8 n° 134 (C-230) 1571", lui, décrit la synthèse d'une mordénite comprenant du fluor à partir d'une mordénite synthétisée en milieu classique (OH⁻). Quant à la demande de brevet EP-A-0.040.104, elle, décrit la synthèse en milieu classique d'une zéolithe dont le diagramme de diffraction est très proche de celui de la mordénite.

L'invention a donc pour objet un procédé de synthèse d'une nouvelle zéolithe crystalline synthétique grâce auquel les inconvénients cités précédemment sont évités et qui confère de plus aux zéolithes selon l'invention des propriétès, particulièrement des propriétés acides, améliorées. Le nouveau type de zéolithe suivant l'invention peut être utilisé notamment en adsorption et en catalyse, notamment en isomérisation des xylènes.

La zéolithe de type mordénite suivant l'invention a (après synthèse) la formule générale approchée suivante :

$$Na_2O, Al_2O_3, x\ SiO_2$$

La zéolithe selon l'invention est notamment caractérisée par :

a) un nombre x compris entre 9 et 30, de préférence entre 9 et 20, de manière encore plus préférée entre 9 et 15 (x étant le rapport molaire $SiO_2/Al_2O_3$),

b) un diagramme de diffraction des rayons X représenté dans le tableau I de la description,

c) une teneur en fluor comprise entre environ 0,005 et 2,0 % en poids, de préférence entre environ 0,01 et 1,5 % en poids, de manière encore plus préférée entre environ 0,01 et 0,5 %.

Elle est également caractérisée par le fait qu'elle a été synthétisée en milieu fluorure.

Cette nouvelle zéolithe de type mordénite selon l'invention présente généralement au moins une dimension des cristaux comprise entre 0,10 et 5 $\mu$m et, de préférence, entre 1 et 2 $\mu$m (1 $\mu$m = $10^{-6}$ mètre).

L'invention concerne aussi un procédé de préparation de ladite zéolithe de type mordénite, qui consiste en ce que :

a) on forme un mélange réactionnel en solution ayant un pH inférieur à environ 10 et comprenant de l'eau, au moins une source de silice, au moins une source d'aluminium, au moins une source d'agent mobilisateur contenant des ions fluorures (F⁻), une source de cations sodium (Na⁺), ledit mélange réactionnel ayant une composition, en termes de rapport molaire, comprise dans les intervalles de valeurs suivants :

| Si/Al | 5-50, de préférence 5-25, |
|---|---|
| F⁻/Si | 0,1-10, de préférence 0,25-8, |
| Na⁺/Si | 0,1-10, de préférence 0,25-8, |
| $H_2O$/Si | 5-25, de préférence 8-25, |

b) on maintient ledit mélange réactionnel à une température de chauffage comprise entre environ 90 et environ 250°C, de préférence entre environ 130 et environ 220°C, jusqu'à ce que l'on obtienne un composé cristallin.

On peut avantageusement chauffer le mélange réactionnel dans un autoclave revêtu intérieurement de polytétrafluoroéthylène (PTFE) entre environ 90 et environ 250°C et, de préférence, entre environ 130 et environ 220°C, pendant une durée qui peut varier de quelques heures à quelques jours (habituellement 8 à 1200 heures) selon la température de réaction retenue, jusqu'à l'obtention d'un solide cristallisé que l'on sépare des eaux-mères généralement par filtration et qui est ensuite lavé par exemple à l'eau distillée.

De manière avantageuse, on peut préparer ledit mélange réactionnel à un pH compris entre environ 4 et environ 10 et, de manière préférée, entre environ 6 et environ 10.

Selon un mode préféré de préparation des zéolithes de type mordénite suivant l'invention, les rapports molaires des constituants du mélange réactionnel sont compris dans les intervalles de valeurs suivants :

| Si/Al | 6,5-15 |
| $F^-$/Si | 0,4-5 |
| $Na^+$/Si | 0,4-5 |
| $H_2O$/Si | 8-22. |

On peut éventuellement ajouter audit mélange réactionnel au moins un sel complémentaire dans un rapport molaire sel complémentaire/$SiO_2$ compris généralement entre 0,1 et 4 et, de préférence, entre 0,2 et 0,5 et/ou au moins un germe de la zéolithe formée selon l'invention dans un rapport pondéral cristal de zéolithe/$SiO_2$ compris généralement entre 0,01 et 0,1 et, de manière préférée, entre 0,02 et 0,03, de telle sorte que la morphologie, la taille des cristaux ainsi que la cinétique de la réaction de cristallisation puissent être avantageusement contrôlées.

On peut travailler avantageusement en milieu agité, ce qui peut permettre de diminuer considérablement le temps de réaction.

Le pH du milieu réactionnel, inférieur à environ 10, peut être obtenu soit directement à partir de l'un ou plusieurs des réactifs mis en oeuvre, soit par l'ajout d'un acide, d'une base, d'un sel acide, d'un sel basique ou d'un mélange tampon complémentaire.

De nombreuses sources de silice peuvent être utilisées. On peut citer notamment les silices sous forme d'hydrogels, d'aérogels, de suspensions colloïdales, ainsi que les silices résultant de la précipitation de solutions de silicates solubles ou de l'hydrolyse d'esters siliciques comme l'ester tétraéthylique de l'acide orthosilicique $Si(OC_2H_5)_4$ ou de complexes comme le fluorosilicate de sodium $Na_2SiF_6$ ou d'ammonium $(NH_4)_2SiF_6$.

Parmi les sources d'aluminium utilisées, on choisira de préférence le chlorure d'aluminium hydraté ($AlCl_3, 6H_2O$), le nitrate d'aluminium nonahydraté ($Al(NO_3)_3, 9H_2O$), le sulfate d'aluminium à 16 molécules d'eau ou le fluorure d'aluminium trihydraté $AlF_3, 3H_2O$. On peut également citer la pseudo-boehmite.

Par ailleurs, au lieu de partir de sources séparées de silice et d'aluminium, on peut également prendre des sources où les deux éléments sont combinés comme par exemple un gel aluminosilicaté fraîchement précipité.

Les anions fluorures $F^-$ peuvent être introduits sous forme de sels de sodium ou d'ammonium ou de métaux alcalins comme par exemple NaF, $NH_4F$, $NH_4HF_2$, ou sous forme d'acide HF ou sous forme de composés hydrolysables pouvant libérer des anions fluorures dans l'eau comme le fluorure de silicium $SiF_4$ ou le fluorosilicate d'ammonium $(NH_4)_2SiF_6$ ou de sodium $Na_2SiF_6$.

Les acides ou sels acides, les bases ou sels basiques ajoutés éventuellement en complément pour amener le pH du milieu réactionnel à la valeur désirée peuvent être choisis parmi les acides courants comme, par exemple, HF, HCl, $HNO_3$, $H_2SO_4$, $CH_3COOH$ ou les sels acides comme, par exemple, $NH_4HF_2$, $KHF_2$, $NaHSO_4$, les bases courantes comme, par exemple, $NaHCO_3$, $CH_3COONa$, $Na_2S$, NaHS ou les mélanges tampons comme, par exemple, ($CH_3COOH$, $CH_3COONa$) ou ($NH_4OH$, $NH_4Cl$).

On peut éventuellement introduire dans la zéolithe de structure mordénite selon l'invention, par des techniques d'échanges ioniques bien connues dans l'art antérieur, au moins un élément de la classification périodique, dont les cations peuvent être préparés en milieu aqueux et choisis par exemple dans la famille constituée par les groupes IIA, IIIA, IB, IIB, IIIB, IVB et VIIIA de la classification périodique des éléments. On citera à titre d'exemple les cations alcalins, alcalino-terreux, les cations de terres rares, $Fe^{II}$, $Fe^{III}$, $Co^{II}$, $Co^{III}$, $Ni^{II}$, $Cu^{II}$, $Zn^{II}$, $Ag^{I}$, $Pt^{II}$.

L'identification des zéolithes de type mordénite suivant l'invention peut se faire de manière aisée à partir de leur diagramme de diffraction des rayons X. Ce diagramme de diffraction peut être obtenu à l'aide d'un diffractomètre en utilisant la méthode classique des poudres avec le rayonnement $K\alpha$ du cuivre. Un étalon interne permet de déterminer précisément les valeurs des angles $2\theta$ associées aux pics de diffraction. Les différentes distances interréticulaires $d_{hkl}$, caractéristiques de l'échantillon, sont calculées à partir de la relation de Bragg. L'estimation de l'erreur de mesure $\Delta(d_{hkl})$ sur $d_{hkl}$ se calcule en fonction de l'erreur absolue $\Delta(2\theta)$ affectée à la mesure de $2\theta$ par la relation de Bragg. En présence d'un étalon interne, cette erreur minimisée et prise couramment égale à $\pm 0,05°$. L'intensité relative $I/I_0$ affectée à

4

chaque valeur de $d_{hkl}$ est estimée à partir de la hauteur du pic de diffraction correspondant. Cette dernière peut également être déterminée à partir d'un cliché obtenu à l'aide d'une chambre de Debye-Scherrer. On utilise souvent une échelle de symboles pour caractériser cette intensité : FF = très forte, F = forte, mF = moyenne à forte, m = moyenne, mf = moyenne à faible, f = faible, ff = très faible.

Le tableau I représente le diagramme de diffraction des rayons X caractéristique des zéolithes de type mordénite selon l'invention. Dans la colonne des $d_{hkl}$ on a représenté les valeurs extrêmes que peuvent prendre les différentes distances interréticulaires $d_{hkl}$. Chacune de ces valeurs doit être affectée de l'erreur de mesure $\Delta(d_{hkl})$ généralement comprise entre ± 0,07 et ± 0,002 suivant la valeur de 2 $\theta$ ($d_{hkl}$ est exprimée en Angström, 1 Å = $10^{-10}$ m).

La zéolithe de structure mordénite selon l'invention peut être utilisée seule ou en mélange avec une matrice au sein d'un catalyseur.

Ladite zéolithe peut par exemple, après synthèse, être mise en forme en utilisant une matrice qui peut être inerte ou active pour la réaction à promouvoir. Les matrices que l'on emploie sont généralement choisies dans le groupe formé par les argiles, les alumines, la silice, la magnésie, la zircone, l'oxyde de titane, l'oxyde de bore et toute combinaison d'au moins deux des composés précités comme la silice-alumine, la silice-magnésie etc... Toutes les méthodes connues d'agglomération et de mise en forme sont applicables, telles que par exemple, l'extrusion, le pastillage, la coagulation en goutte etc...

Le catalyseur possède alors une teneur pondérale en zéolithe de type mordénite selon l'invention généralement comprise entre 20 et 99,5 %, de préférence entre 40 et 95 % et une teneur pondérale en matrice généralement comprise entre 0,5 et 80 %, de préférence entre 5 et 60 %.

Le catalyseur contenant la zéolithe de structure mordénite selon l'invention peut renfermer en outre une fonction hydrogénante ou déshydrogénante, constituée en général par au moins un métal ou/et composé de métal choisi parmi les groupes IA, VIB (Cr, Mo, W) et VIII de la classification périodique des éléments, par exemple le platine, le palladium ou/et le nickel.

La zéolithe de structure mordénite selon l'invention peut être mise en oeuvre, seule ou en mélange avec une matrice (et généralement une fonction hydrogénante ou déshydrogénante) au sein d'un catalyseur, dans un procédé d'isomérisation des xylènes. Les conditions opératoires dudit procédé sont habituellement les suivantes :

- température comprise entre 240 et 600°C, de préférence entre 350 et 510°C,
- pression comprise entre 0,5 et 100 bars, de préférence entre 2 et 30 bars,
- vitesse spatiale (pph), en masse de charge par unité de charge de catalyseur et par heure, comprise entre 0,5 et 200 $(heure)^{-1}$, de préférence entre 2 et 100 $(heure)^{-1}$
- rapport molaire hydrogène sur hydrocarbures de la charge ($H_2$/HC) compris entre 0,5 et 12, de préférence entre 2 et 6.

TABLEAU I

| 2 $\theta$(°) | $d_{hkl}$ (Å) | I/Io |
|---|---|---|
| 6,38 | 13,84 | mF |
| 8,50 | 10,39 | mf |
| 9,60 | 9,21 | F |
| 13,38 | 6,61 | mF |
| 13,70 | 6,46 | m |
| 14,35 | 6,17 | ff |
| 15,10 | 5,86 | f |
| 19,48 | 4,55 | mF |
| 20,72 | 4,28 | ff |
| 22,15 | 4,01 | F |
| 23,00 | 3,86 | ff |
| 23,50 | 3,78 | f |
| 24,30 | 3,66 | ff |
| 25,55 | 3,48 | FF |
| 26,20 | 3,398 | F |
| 27,65 | 3,223 | mF |
| 30,75 | 2,905 | m |
| 32,6 | 2,744 | ff |
| 33,08 | 2,706 | ff |
| 34,93 | 2,566 | ff |
| 35,55 | 2,523 | f |
| 40,39 | 2,231 | ff |
| 44,3 | 2,088 | f |

Les exemples suivants illustrent l'invention sans en limiter la portéé.

EXEMPLE 1

0,93 g de NaF (0,0224 mole) sont dissous à température ambiante dans 9,36 g d'eau (0,52 mole) ; à cette solution sont alors ajoutés sous agitation et successivement :

3,73 g de "tixolex 28 Na$^+$" (rapport Si/Al = 7)
(0,052 mole SiO$_2$ et 7,43.10$^{-3}$ mole Al)

et environ 60 mg de mordénite-Na$^+$ broyée (environ 2 % en poids de la silice engagée).

Après une dizaine de minutes d'agitation, le mélange est transféré dans un autoclave de 75 ml muni d'un chemisage interne en téflon. L'autoclave est alors placé directement à l'étuve pendant 7 jours à 200°C.

Les rapports molaires du mélange réactionnel sont les suivants :

Si/Al = 7 ; F$^-$/Si = 0,43 ; Na$^+$/Si = 0,57 ; H$_2$O/Si = 10.

Le pH du mélange réactionnel est de 9,5.

Après la synthèse le solide est recueilli par filtration, lavé à l'eau distillée, séché à l'étuve à 80°C pendant 24 heures puis analysé par diffraction des rayons X.

Le produit est complètement cristallisé sous forme de mordénite, aucune impureté n'étant décelable sur le diffractogramme. Les cristaux se présentent sous forme de "bâtonnets", de 1 à 2$\mu$m sur quelques dizième de micron de large.

La teneur pondérale en fluor du produit est de 0,16 %.

Le rapport molaire SiO$_2$/Al$_2$O$_3$ du produit, déterminé chimiquement est de 13,6. Compte tenu du poids de mordénite obtenue, soit environ 3 g, le rendement de la réaction est élevé et proche de 80 % par rapport au poids de silice engagée.

EXEMPLE 2 Identique exemple 1, sauf 1,75 g NaF engagés (0,0416 mole) (rapport molaire F$^-$/Si = 0,8) ; la teneur pondérale en fluor du produit obtenu est de 0,20 %.

EXEMPLE 3 Identique exemple 1, sauf 5,24 g NaF engagés (0,125 mole) (rapport molaire F⁻/Si = 2,4) ; la teneur pondérale en fluor du produit obtenu est de 0,25 %.

EXEMPLE 4 Identique exemple 1, sauf 10,5 g Naf engagés (0,250 mole) (rapport molaire F⁻/Si = 4,8).

EXEMPLE 5 Identique exemple 1, sauf en absence de NaF (rapport molaire F⁻ /Si = 0).

| Tableau récapitulatif exemples 1 à 5 | | | | | |
|---|---|---|---|---|---|
| Exemple | Rapport molaire F⁻/Si dans le mélange réactionnel | Produit obtenu | | | |
| | | Nature | Rapport Molaire $SiO_2/Al_2O_3$ | Masse (g) | Taille cristaux ($\mu$m) |
| 1 | 0,43 | 100 % cristallisé mordénite | 13,6 | 3 | 1-2 |
| 2 | 0,80 | 100 % cristallisé mordénite | 13,6 | 2,8 | 2 |
| 3 | 2,40 | 100 % cristallisé mordénite | 13,2 | 3,1 | 1 |
| 4 | 4,80 | 100 % cristallisé mordénite | 13,4 | 3,2 | 1-2 |
| 5 | 0 | gel | - | - | - |

EXEMPLE 6

Les conditions de synthèse sont identiques à celles de l'exemple 1, sauf que 2,62g de NaF (0,0624 mole) sont utilisés (rapport molaire F⁻/Si = 1,2) et que le mélange réactionnel est porté à 170°C pendant 14 jours. Le pH du mélange réactionnel est voisin de 9,5.

On obtient environ 3,1 g de mordénite bien cristallisée, sous forme de cristaux de taille comprise entre environ 1 et 2$\mu$m.

EXEMPLE 7

Les conditions de synthèse sont identiques à celles de l'exemple 1,sauf que 2,62g de NaF (0,0624 mole) sont utilisés (rapport molaire F⁻/Si = 1,2) et que la température de synthèse est de 135°C et la durée de 21 jours. Le pH du mélange réactionnel est de 9,5 environ. Le produit obtenu est complètement cristallisé sous forme de mordénite, la taille des cristaux étant comprise entre 1 et 2$\mu$m.

EXEMPLE 8

Les conditions de synthèse sont identiques à celles de l'exemple 1, sauf que 2,62g de NaF (0,0624 mole) sont utilisés (rapport molaire F⁻/Si = 1,2) et que la source de silicium est de type aérosil (Degussa). 3,12 g d'aérosil sont utilisés, soit 0,052 mole de silicium. La source d'aluminium est de type pseudo-boehmite. 0,60 g de pseudo-boehmite sont utilisés, soit $7,5 \cdot 10^{-3}$ mole d'aluminium. Le rapport molaire $H_2O/Si$ = 20. Le pH du mélange réactionnel est de 7 environ. Après réaction le pH est de 9,5 environ. Le produit obtenu est complètement cristallisé sous forme de mordénite ; les cristaux ont une longueur d'environ 2$\mu$m.

EXEMPLE 9

Les conditions de synthèse sont identiques à celles de l'exemple 1, mis à part le fait que la durée de synthèse est de 4 jours à la température de 200°C. Le pH du mélange réactionnel est de 9,5 environ. Après synthèse) le pH est de 9,5 également.

La masse de produit recueilli est voisine de 3 g ; le gel est entièrement transformé en mordénite (masse obtenue : 3 g ;taille des cristaux : 1-2 $\mu$m)

EXEMPLE 10

63 g de NaF, soit 1,5 mole de NaF, sont dissous à température ambiante : à cette solution on ajoute sous agitation 134,19 g de Tixolex 28 (rapport Si/Al = 7) soit 1,87 mole de $SiO_2$ et 0,267 mole d'aluminium et environ 2,23 g de mordénite-$Na^+$ broyée (soit environ 2 % en poids de la silice engagée).

Après 30 minutes d'agitation, le mélange réactionnel, de pH = 9,7, est transféré dans des autoclaves de 75 ml revêtu intérieurement de téflon.

Le mélange réactionnel a la composition molaire suivante :

Si/Al = 7 ; F/Si = 0,8 ; $H_2O$/Si = 10.

Le mélange réactionnel est porté à 200°C pendant 5 jours. Après réaction, le pH est de 10. Après filtration et lavage à l'eau distillée, le solide est séché à l'étuve à 80°C pendant 24 h puis analysé par diffraction des rayons X.

Le produit est cristallisé sous forme de mordénite. Son rapport molaire $SiO_2$/$Al_2O_3$, déterminé chimiquement, est de 14,5.

EXEMPLE 11 Préparation d'un catalyseur.

La mordénite obtenue à l'exemple 10 est ensuite intimement mélangée avec de l'alumine sur laquelle on a dispersé 0,45 % en poids de platine. Le support est constitué par le mélange mordénite-alumine contenant 40 % en poids d'alumine. La teneur pondérale en platine du catalyseur final est de 0,18 %.

Le catalyseur ainsi fabriqué est ensuite mis en forme par pastillage, calciné sous air jusqu'à 550°C durant 2 heures et réduit sous hydrogène à 450°C pendant 3 heures.

EXEMPLE 12

Le catalyseur décrit dans l'exemple 11 est mis en oeuvre dans la réaction d'isomérisation de l'orthoxylène, à une température de 410°C, sous une pression de 12 bars, avec une vitesse spatiale (pph) de 35 $(heure)^{-1}$ et un rapport $H_2$/HC de 4 environ. Les performances de ce catalyseur, et de celui qui sera décrit dans l'exemple suivant, reportées dans le tableau II, sont définies par :

Approche à équilibre de l'o.x (calculée sur le mélange des 3 xylènes) =

$$\frac{\text{Masse d'o.x dans la charge - Masse d'o.x. dans la recette}}{\text{Masse d'o.x dans la charge - Masse d'o.x à l'équilibre}} \times 100$$

Approche à l'équilibre du p.x (calculée sur le mélange des 3 xylènes) =

$$\frac{\text{Masse de p.x dans la recette}}{\text{Masse de p.x à l'équilibre}} \times 100$$

Rendement en $C_8$ aromatiques et $C_8$ naphténiques =

$$\frac{\text{Masse de } C_8 \text{ arom. dans la recette + Masse de } C_8 \text{ napht. dans la recette}}{\text{Masse totale de recette}} \times 100$$

% de dismutation = (% poids de $C_9$ dans la recette)$(1+ \frac{\text{Masse Molaire de } C_7}{\text{Masse Molaire de } C_9})$

+( % poids de $C_{10}$ dans la recette) $(1 + \frac{\text{Masse molaire de } C_6}{\text{Masse molaire de } C_{10}})$

% de dealkylation = (% poids de $C_6$ dans la recette) -
(% poids de $C_{10}$ dans la recette)$(\frac{\text{Masse molaire de } C_6}{\text{Masse molaire de } C_{10}})$+
(% poids de $C_7$ dans la recette) -
(% poids de $C_9$ dans la recette) $(\frac{\text{Masse molaire de } C_7}{\text{Masse molaire de } C_9})$

% craquage = % poids dans la recette de $(C_3 + C_4 + C_5)$

(avec o.x = o-xylène et p.x = p-xylène)

EXEMPLE 13 (comparatif)

On prépare un catalyseur à partir d'une mordénite larges pores sous forme de poudre référencée Zeolon 100 Na de la société Norton. La poudre est soumise à 3 échanges avec une solution de nitrate d'ammonium 4M. Chaque échange est effectué pendant $\frac{1}{2}$ heure, à la température de reflux.

Après le dernier échange, le produit est lavé à l'eau pendant 20 mn, filtré et séché en étuve à 120°C. Son rapport molaire $SiO_2/Al_2O_3$ est égal à 13 et sa teneur en sodium à 75 ppm.

La préparation du catalyseur et le test catalytique sont réalisés dans les conditions décrites dans les exemples 11 et 12.

Les résultats catalytiques figurent dans le tableau II.

La mordénite synthétisée en milieu fluorure selon l'invention conduit à un catalyseur plus sélectif, les réactions secondaires et en particulier les réactions de dismutation et de craquage sont considérablement inhibées. Ce résultat pourrait être interprété par une répartition des atomes d'aluminium différente dans les deux solides.

EP 0 427 579 B1

TABLEAU II

| Catalyseur | A | B |
|---|---|---|
| AEQ o.x (*) | 65,2 | 66,0 |
| Rdt $C_8$ aro + napht | 94,1 | 94,5 |
| AEQ p.x(**) | 44,6 | 50 |
| % dismutation | 1,25 | 4,4 |
| % dealkylation | 0,06 | 0,15 |
| % craquage | 0,2 | 0,5 |

(*) Approche à l'équilibre de l'orthoxylène
(**) Approche à l'équilibre du paraxylène

**Revendications**

1. Procédé de préparation d'une zéolithe cristalline synthétique de type mordénite de caractéristiques suivantes :
   - une formule générale approchée suivante :

   $Na_2O, Al_2O_3, xSiO_2$

   où x est un nombre compris entre 9 et 30, et
   - un diagramme de diffraction des rayons X représenté dans le tableau suivant

| $2\theta(°)$ | $d_{hkl}$ (Å) | I/Io |
|---|---|---|
| 6,38 | 13,84 | mF |
| 8,50 | 10,39 | mf |
| 9,60 | 9,21 | F |
| 13,38 | 6,61 | mF |
| 13,70 | 6,46 | m |
| 14,35 | 6,17 | ff |
| 15,10 | 5,86 | f |
| 19,48 | 4,55 | mF |
| 20,72 | 4,28 | ff |
| 22,15 | 4,01 | F |
| 23,00 | 3,86 | ff |
| 23,50 | 3,78 | f |
| 24,30 | 3,66 | ff |
| 25,55 | 3,48 | FF |
| 26,20 | 3,398 | F |
| 27,65 | 3,223 | mF |
| 30,75 | 2,905 | m |
| 32,6 | 2,744 | ff |
| 33,08 | 2,706 | ff |
| 34,93 | 2,566 | ff |
| 35,55 | 2,523 | f |
| 40,39 | 2,231 | ff |
| 44,3 | 2,088 | f |

   - une teneur en fluor comprise entre environ 0,005 et 2.0 % en poids,
   caractérisé en ce que :
   a) on forme un mélange réactionnel en solution ayant un pH inférieur à 10 et comprenant de l'eau, au moins une source de silice, au moins une source d'aluminium, au moins une source d'agent mobilisateur contenant des ions fluorures, une source de cations sodium, ledit mélange réactionnel ayant une composition, en termes de rapport molaire, comprise dans les intervalles de valeurs

10

suivants :

| | |
|---|---|
| Si/Al | 5-50 |
| $F^-$/Si | 0,1-10 |
| $Na^+$/Si | 0,1-10 |
| $H_2O$/Si | 5-25, |

b) on maintient ledit mélange réactionnel à une température de chauffage comprise entre environ 90 et environ 250° C jusqu'à ce que l'on obtienne un composé cristallin.

2. Procédé selon la revendication 1 dans lequel, dans l'étape a), ledit mélange réactionnel a une composition, en termes de rapport molaire, comprise dans les intervalles de valeurs suivants :

| | |
|---|---|
| Si/Al | 5-25 |
| $F^-$/Si | 0,25-8 |
| $Na^+$/Si | 0,25-8 |
| $H_2O$/Si | 8-25, |

3. Procédé selon l'une des revendications 1 ou 2 dans lequel, dans l'étape a), ledit mélange réactionnel a une composition, en termes de rapport molaire, comprise dans les intervalles de valeurs suivants :

| | |
|---|---|
| Si/Al | 6,5-15 |
| $F^-$/Si | 0,4-5 |
| $Na^+$/Si | 0,4-5 |
| $H_2O$/Si | 8-22, |

4. Procédé selon l'une des revendications 1 à 3 dans lequel, dans l'étape b), on maintient ledit mélange réactionnel à une température de chauffage comprise entre 130 et 220° C jusqu'à ce que l'on obtienne un composé cristallin.

**Claims**

1. A process for preparing a synthetic crystalline zeolite of the mordenite type characterized by :
   - the following approximate general formula :

   $Na_2O$, $Al_2O_3$, $xSiO_2$

   where x is a number ranging from 9 to 30, and
   - an X-ray diffraction diagram shown in the following table.

| 2 $\theta$ (°) | $d_{hkl}$ (Å) | I/Io |
|---|---|---|
| 6,38 | 13,04 | mF |
| 8,50 | 10,39 | mf |
| 9,60 | 9,21 | F |
| 13,38 | 6,61 | mF |
| 13,70 | 6,46 | m |
| 14,35 | 6,17 | ff |
| 15,10 | 5,86 | f |
| 19,48 | 4,55 | mF |
| 20,72 | 4,28 | ff |
| 22,15 | 4,01 | F |
| 23,00 | 3,86 | ff |
| 23,50 | 3,78 | f |
| 24,30 | 3,66 | ff |
| 25,55 | 3,48 | FF |
| 26,20 | 3,398 | F |
| 27,65 | 3,223 | mF |
| 30,75 | 2,905 | m |
| 32,6 | 2,744 | ff |
| 33,08 | 2,706 | ff |
| 34,93 | 2,566 | ff |
| 35,55 | 2,523 | f |
| 40,39 | 2,231 | ff |
| 44,3 | 2,088 | f |

• a fluorine content ranging from about 0,005 to 2,0 %

characterized in that it comprises :

a) forming a dissolved reaction mixture with a pH value lower than about 10 and comprising water, at least one silica source, at least one aluminum source, at least one source of a mobilizing agent containing fluoride ions, one source of sodium cations, said reaction mixture having a composition, expressed in molar ratio, ranging between the following values :

| | |
|---|---|
| Si/Al | 5-50 |
| $F^-$/Si | 0,1-10 |
| $Na^+$/Si | 0,1-10 |
| $H_2O$/Si | 5-25, |

b) maintaining said reaction mixture at a heating temperature ranging from about 90 to about 250° C until a crystalline compound is obtained.

2. A process according to claim 1, wherein, in stage a), said reaction mixture has a composition, expressed in molar ratio, ranging between the following values :

| | |
|---|---|
| Si/Al | 5-25 |
| $F^-$/Si | 0,25-8 |
| $Na^+$/Si | 0,25-8 |
| $H_2O$/Si | 5-25, |

3. A process according to any one of claims 1 or 2 wherein, in stage a), said reaction mixture has a composition, expressed in molar ratio, ranging between the following values :

12

| Si/Al | 6.5-15 |
|---|---|
| $F^-/Si$ | 0,4-5 |
| $Na^+/Si$ | 0,4-5 |
| $H_2O/Si$ | 8-22, |

4. A process according to any one of claims 1 to 3 wherein, in stage b), said reaction mixture is maintained at a heating temperature ranging from about 130 to 220° C until a crystalline compound is obtained.

**Patentansprüche**

1. Verfahren zur Herstellung eines synthetischen kristallinen Zeoliths vom Mordenit-Typ mit folgenden Eigenschaften:
   - eine folgende allgemeine angenäherte Formel:

   $Na_2O, Al_2O_3, xSiO_2$,

   wobei x eine Zahl zwischen 9 und 30 ist, und
   - ein Röntgenbeugungsdiagramm, das in der folgenden Tabelle dargestellt ist:

| $2\theta$ (°) | $d_{hkl}$ (Å) | I/Io |
|---|---|---|
| 6,38 | 13,84 | mF |
| 8,50 | 10,39 | mf |
| 9,60 | 9,21 | F |
| 13,38 | 6,61 | mF |
| 13,70 | 6,46 | m |
| 14,35 | 6,17 | ff |
| 15,10 | 5,86 | f |
| 19,48 | 4,55 | mF |
| 20,72 | 4,28 | ff |
| 22,15 | 4,01 | F |
| 23,00 | 3,86 | ff |
| 23,50 | 3,78 | f |
| 24,30 | 3,66 | ff |
| 25,55 | 3,48 | FF |
| 26,20 | 3,398 | F |
| 27,65 | 3,223 | mF |
| 30,75 | 2,905 | m |
| 32,6 | 2,744 | ff |
| 33,08 | 2,706 | ff |
| 34,93 | 2,566 | ff |
| 35,55 | 2,523 | f |
| 40,39 | 2,231 | ff |
| 44,3 | 2,088 | f |

   - einen Gehalt an Fluor einschließlich zwischen etwa 0,005 und 2.0 Gew.-%,

   dadurch gekennzeichnet, daß:
   a) man ein Reaktionsgemisch in Lösung bildet, das einen pH-Wert von kleiner als 10 aufweist und Wasser, wenigstens eine Siliziumdioxidquelle, wenigstens eine Aluminiumquelle, wenigstens eine Mobilisierungswirkstoffquelle, welche Floridionen enthält, eine Natriumkationenquelle umfaßt, wobei das Reaktionsgemisch eine Zusammensetzung, bezogen auf das Molverhältnis, aufweist, welche in den folgenden Werteintervallen enthalten ist:

| Si/Al | 5-50 |
|---|---|
| $F^-$/Si | 0,1-10 |
| $Na^+$/Si | 0,1-10 |
| $H_2O$/Si | 5-25, |

b) man das Reaktionsgemisch auf einer Heiztemperatur einschließlich zwischen etwa 90 und etwa 250° C hält, bis man eine kristalline Zusammensetzung erhält.

2. Verfahren nach Anspruch 1, bei welchem das Reaktionsgemisch im Schritt a) eine Zusammensetzung, bezogen auf das Molverhältnis, aufweist, welche in den folgenden Werteintervallen enthalten ist:

| Si/Al | 5-25 |
|---|---|
| $F^-$/Si | 0,25-8 |
| $Na^+$/Si | 0,25-8 |
| $H_2O$/Si | 8-25. |

3. Verfahren nach einem der Ansprüche 1 oder 2, bei welchem das Reaktionsgemisch im Schritt a) eine Zusammensetzung, bezogen auf das Molverhältnis, aufweist, welche in den folgenden Werteintervallen enthalten ist:

| Si/Al | 6,5-15 |
|---|---|
| $F^-$/Si | 0,4-5 |
| $Na^+$/Si | 0,4-5 |
| $H_2O$/Si | 8-22. |

4. Verfahren nach einem der Ansprüche 1 bis 3, bei welchem man das Reaktionsgemisch im Schritt b) auf einer Heiztemperatur einschließlich zwischen 130 und 220° C hält, bis man eine kristalline Zusammensetzung erhält.